# FASCICULE DE BREVET EUROPEEN

(11) **EP 0 633 791 B1**
(45) Date de publication et mention de la délivrance du brevet: **18.12.1996**
(21) Numéro de dépôt: 93907907.5
(22) Date de dépôt: 30.03.1993
(51) Int. Cl.: A61M 5/20, A61M 5/46, A61M 5/48

(54) **APPAREIL D'INJECTION MEDICALE OU VETERINAIRE**
MEDIZINISCHER ODER VETERINAERER INJEKTIONSAPPARAT
MEDICAL OR VETERINARY INJECTION APPARATUS

(30) Priorité: 31.03.1992 FR 9203870
(43) Date de publication de la demande: 18.01.1995
(73) Titulaire: MATEF, F-92500 Rueil Malmaison (FR)
(72) Inventeur: TRAPP, Claude, F-92500 Rueil-Malmaison (FR); CHARTON, Jean-Pierre, F-21000 Dijon (FR)
(74) Mandataire: Hud, Robert
(86) Numéro de dépôt international: FR9300315
(87) Numéro de publication internationale: WO9319794

(56) Documents cités:
- EP-A- 0 238 378
- FR-A- 2 401 667
- FR-A- 2 567 760
- US-A- 3 768 472

## Description

L'invention concerne des appareils médicaux ou vétérinaires et s'applique plus particulièrement à l'injection de liquides dans des tissus vivants au moyen d'une aiguille creuse qui conduit le liquide, sous faible pression, jusqu'à son extrémité implantée dans le derme ou dans la chair. Un autre secteur concerné est celui des traitements de denrées alimentaires.

On connaît diverses réalisations d'appareils qui permettent de suppléer à la main du praticien pour l'implantation de l'aiguille et pour l'injection du liquide. Ces appareils ont pour but d'éviter au patient des douleurs à l'implantation ou lors de l'injection, ainsi que d'éviter de la fatigue à l'opérateur. Toutefois, jusqu'ici, ces appareils n'ont pas donné satisfaction, étant trop lourds, trop coûteux et insuffisamment prémunis contre des risques de transmission d'infection d'un sujet à un autre.

Par exemple, le brevet des Etats-Unis d'Amérique n° 4 790 823 délivré le 13 décembre 1988 aux noms de CHARTON et GASQUET décrit un appareil comportant une pièce à main reliée par une canalisation souple à une console posée sur le sol, la console fournissant à la pièce à main de l'air comprimé ou du courant électrique et même éventuellement le liquide à injecter sous pression. Celui-ci est issu d'une seringue dont le piston est poussé par une vis actionnée par un micromoteur électrique. Toutefois la console est encombrante et coûteuse et la pièce à main, bien que légère en soi, lui est reliée par une conduite d'air comprimé et éventuellement un fil électrique, ce qui gêne sa manipulation.

Le brevet français n° 78.08827 (publié sous le n° 2 401 667), au nom de ISLAND S.R.L., décrit un dispositif analogue, où le piston de la seringue, équipée de l'aiguille et portée par la pièce à main, est poussé par un vérin pneumatique alimenté en gaz comprimé par un tuyau souple relié par un raccord à démontage rapide à un détendeur qui équipe un récipient de gaz sous pression distinct de la pièce à main. Ici encore la tuyauterie gène la manipulation de la pièce à main qui n'est pas autonome lors des injections. En outre la catapulte, portant aiguille et seringue, est assez lourde, ce qui nécessite une assez forte presion pour alimenter le vérin et donc une forte consommation d'air comprimé limitant l'autonomie du dispositif. D'autre part le vérin pneumatique, qui pousse le piston de la seringue pour injecter le liquide, reçoit une pression réglée par un orifice à section variable, ce qui soumet la vitesse d'injection, donc le volume injecté, à la dextérité de l'opérateur.

Le brevet français n° 84.11336 (publié sous le n° 2 567 760), aux noms de HIBON et KABLA, décrit une pièce à main en forme de pistolet autonome, dont la poignée contient une bouteille amovible de gaz sous pression. Ici encore la catapulte porte l'ensemble aiguille et seringue, assez lourd, et d'autre part la poussée du piston de la seringue est assurée par le même vérin que celui de la catapulte ce qui ne permet pas de régler la pression d'injection.

La présente invention a pour objet de remédier aux inconvénients présentés par les dispositifs connus et elle propose à cet effet un appareil d'injection comportant une autonomie suffisante pour la pratique médicale, permettant une implantation et une injection précises et indolores et diminuant la fatigue de l'opérateur, tout en se prêtant à une aseptie rigoureuse.

Pour ce faire l'appareil selon l'invention est constitué de façon connue par une pièce à main contenant d'une part un vérin pneumatique, conçu pour pousser à l'encontre d'un ressort de rappel une catapulte portant une aiguille d'injection, et d'autre part une seringue amovible contenant le liquide et connectée à l'aiguille avec en outre un organe pneumatique pour pousser un piston mobile dans la seringue, le vérin et l'organe pneumatique étant alimentés par un même récipient équipé d'une part d'un détendeur délivrant un gaz à pression constante au vérin et d'autre part d'un orifice à section variable délivrant du gaz sous pression réglable à l'organe pneumatique, et il se caractérise en ce que l'orifice à section variable fait partie d'un deuxième détendeur qui délivre du gaz à pression stable et réglable à l'organe pneumatique, et en ce que la pièce à main contient le récipient, le détendeur à pression constante et le deuxième détendeur.

De préférence, l'appareil a la forme d'un pistolet dont la poignée constitue le récipient de gaz comprimé pour avoir le volume maximal disponible. L'air comprimé est produit par un petit compresseur indépendant raccordé pour le remplissage du récipient au moyen d'un raccord rapide qui peut être de tout type connu.

De préférence encore l'appareil selon l'invention utilise, selon l'enseignement du brevet des Etats-Unis d'Amérique N° 4 790 823 précédemment cité, une catapulte légère et précise portant uniquement l'aiguille, celle-ci étant reliée par un tube mince flexible à une seringue fixée à la pièce à main, l'ensemble aiguille et seringue n'étant en outre pas séparable et étant jetable après utilisation.

Pour diminuer au maximum le poids et l'encombrement de l'appareil, l'organe pneumatique de poussée du liquide est de préférence une chambre de pression sur laquelle la seringue est fixée avec étanchéité, de sorte que la pression pneumatique pousse directement le piston dans la seringue.

Pour bien faire comprendre l'appareil selon l'invention on en décrira ci-après, à titre d'exemple sans caractère limitatif, une forme d'exécution préférée en référence au dessin schématique annexé dans lequel :
la figure 1 est une vue en coupe verticale longitudinale d'un appareil d'injection selon la présente invention ;
la figure 2 est une vue de dessus, en coupe partielle, de l'appareil de la figure 1 ;
les figures 3a et 3b sont des vues latérales d'une seringue équipée d'un piston libre et munie de son outillage de remplissage, ledit piston étant représenté respectivement à la graduation zéro de la seringue et en position de remplissage complet de la seringue ; et
la figure 4 est une vue en coupe longitudinale, agrandie, d'une variante du dispositif de pincement.

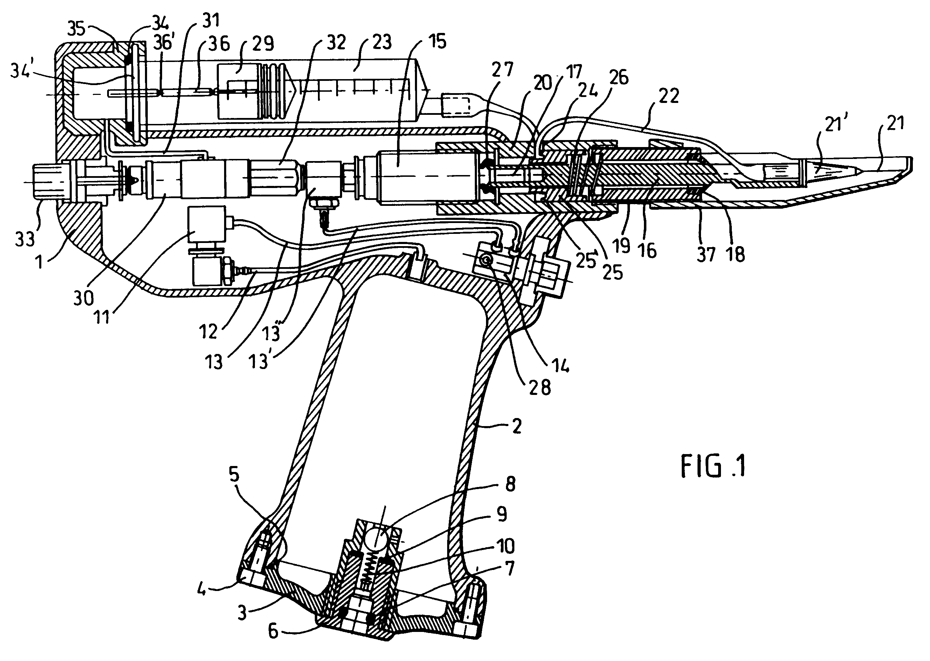

En référence plus particulièrement à la figure 1, le dispositif selon l'invention est constitué d'une pièce à main formée d'un corps 1 prolongé par une poignée 2, celle-ci formant un récipient fermé par un couvercle 3 retenu par des vis telles que 4 avec un joint d'étanchéité 5.

Le couvercle 3 comporte un raccord de remplissage 6 avec un joint 7 destiné à coopérer avec un tube de remplissage non figuré et issu d'une source d'air comprimé à 9 bar par exemple. Le raccord 6 est équipé d'un clapet anti-retour constitué d'une bille 8 que la pression interne d'air comprimé peut appliquer sur un siège 9 par effet autoclave dès le retrait du tube de remplissage. Un petit ressort 10 est prévu pour repousser depuis l'extérieur la bille 8 à l'encontre de toute pression interne inférieure à une pression minimale, déterminée par celle au dessous de laquelle le fonctionnement du dispositif ne serait pas satisfaisant, par exemple au dessous de 2,5 bar. Il produit alors une vidange du récipient, indiquant par là-même la nécessité de le remplir à nouveau.

Le corps 1 contient un premier détendeur 11 relié directement au récipient 2 par un conduit 12, pour débiter de l'air à une pression par exemple de 2,5 bar par un conduit 13. Une vanne à trois voies 14 branchée sur ce conduit 13 et ouverte par actionnement d'un bouton poussoir envoie, par un conduit 13' et un raccord 13'', l'air dans le vérin 15 d'une catapulte 16. Celle-ci est portée par la tige 17 du vérin 15 et par un joint à lèvre 18 encastré à l'extrémité d'un guide 19 fileté extérieurement et vissé dans un tube de liaison 20 fixé au corps 1 et portant à son autre extrémité le vérin 15. Celui-ci,représenté non coupé, est fileté extérieurement et contient un ressort de rappel. Le joint à lèvre 18 évite la pollution de l'intérieur du tube de liaison 20 par des suintements de liquide d'injection qui pourraient retomber le long de l'aiguille d'injection 21.

Cette aiguille 21 est scellée sur un embout 21' lui-même maintenu sur un berceau ouvert qui prolonge la catapulte 16 et qui est assez élastique pour assurer ce maintien. Par l'ouverture du berceau passe un tube de liaison 22, lui-même fixé à une extrémité sur l'embout 21' et à l'autre extrémité sur une seringue graduée 23 contenant le liquide à injecter. Toutes ces pièces sont livrées après aseptie et sont à jeter après usage, sans les séparer.

Le tube 22, appelé catheter, est mince et souple. L'opérateur, après installation de l'aiguille 21 et de la seringue 23, fait pénétrer ce tube 22 dans une fente 24 entaillant transversalement le tube de liaison 20. A l'intérieur de celui-ci et autour de la catapulte 16 est placé un anneau mobile 25, qui est contraint vers la fente 24 par un ressort 26, de sorte qu'il joue le rôle d'une pince pouvant aplatir le tube 22, et donc empêcher l'écoulement du liquide à injecter de la seringue 23 à l'aiguille 21. Lorsque le vérin 15 est actionné, un poussoir 27 formé d'une rondelle serrée entre la tige 17 et la catapulte 16 vient buter contre l'anneau mobile 25 en le repoussant à l'encontre du ressort 26. Le pincement du tube 22 se trouve ainsi supprimé en fin de course de la catapulte 16 qui a implanté l'aiguille 21 à l'endroit voulu, et l'injection du liquide se produit alors.

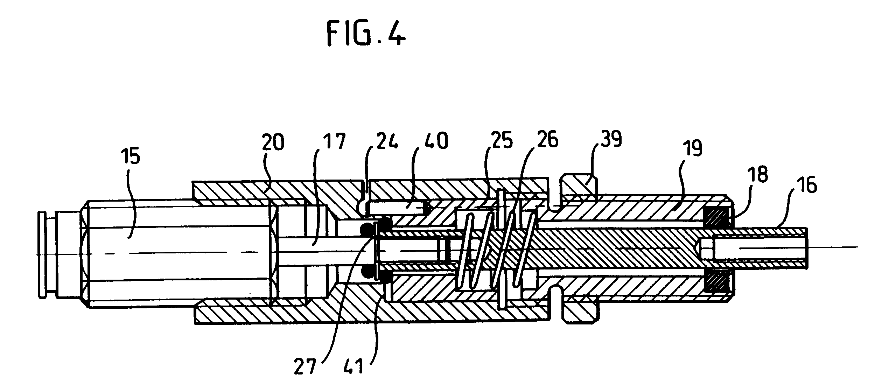

Lorsqu'on relâche la vanne 14, celle-ci met à l'air libre le vérin 15 qui revient en position rétractée sous l'action de son ressort interne. L'anneau mobile 25, repoussé par son ressort 26, revient pincer le tube 22, ce qui arrête l'injection avant que l'aiguille 21 soit retirée. L'anneau mobile 25 comporte à la périphérie de sa face de pincement une saillie étroite 25' pour aplatir le tube 22 sur une faible surface en permettant de diminuer l'effort du ressort 26 et donc la pression dans le vérin 15, au bénéfice de l'autonomie du dispositif. Le poussoir 27 est garni de tores en caoutchouc qui amortissent les chocs contre l'anneau mobile 25 et contre la face du vérin 15, ce qui diminue le bruit de fonctionnement. A la figure 4 qui montre une variante du dispositif de pincement, on retrouve le vérin 15, le tube de liaison 20 avec sa fente 24, l'anneau mobile 25 formant pièce de pincement, le ressort 26, la butée 27 fixée à la tige du vérin sur laquelle est vissée la catapulte 16, celle-ci coulissant dans le joint 18 porté par le guide 19. Ici l'anneau 25 ne comporte pas de saillie périphérique d'aplatissement, mais il est prolongé par une tige 40. Cette tige est guidée à faible jeu dans un épaulement 41 du tube de liaison 20, pour pénétrer dans la gorge 24 laquelle ne débouche pas à l'intérieur de ce tube.

L'appui de la tige 40 sur le cathéter préalablement introduit au fond de la fente 24 est alors ponctuel ce qui permet l'emploi d'un ressort 26 encore moins fort, donc d'une pression pneumatique moindre dans le vérin 15.

On notera que la fente 24 comporte de préférence une largeur d'entrée un peu moindre que le diamètre du cathéter, que l'on y fait pénétrer par étirement, alors que son fond est un perçage transversal au diamètre du cathéter ce qui permet de vérifier que celui-ci est en bonne position en face de la tige 40, car il peut alors coulisser librement dans ce perçage.

Le fond de la fente 24 ne débouche pas dans l'alésage du tube 20, de sorte que la fente et le cathéter restent à l'extérieur et qu'ainsi aucune pollution externe ne peut suinter à l'intérieur du mécanisme.

La mise à l'air libre du vérin 15 peut être ralentie par un diaphragme 28 porté par la vanne 14, pour que le retrait de l'aiguille 21 soit progressif en permettant une répartition du liquide le long du trajet. Ce diaphragme est de préférence réglable, par tout moyen connu approprié, et il peut consister par exemple en une vis-pointeau traversant la paroi du corps 1.

La mise en pression du liquide à injecter résulte d'un organe pneumatique de poussée sur le piston 29 contenu dans la seringue 23. Cet organe est alimenté à partir du raccord 13'' par un détendeur 30 débitant avec une pression stable et réglable dans une canalisation 31. Ce réglage dépend en particulier de la viscosité du liquide à injecter et se fait par un bouton moleté 33. On prévoit éventuellement l'interposition d'un clapet anti-retour 32.

Au lieu d'employer un vérin pour pousser le piston 29, on utilise de préférence selon l'invention la poussée directe sur ce piston de l'air à pression stable et réglable issu du détendeur 30. A cet effet la seringue 13 est pressée contre un joint d'étanchéité 34 et retenue par sa collerette 34' contre une chambre de pression 35 inserrée dans le corps 1.

On a représenté, à la figure 1, une forme de réalisation d'un piston 29 adapté à ce mode de poussée pneumatique. Au lieu d'une tige de piston apte à être poussée manuellement, on utilise une tige 36 fixée au piston mais mince pour servir uniquement à la traction du piston pour le remplissage de la seringue. En outre cette tige 36 comporte des gorges de fragilisation 36' faisant des amorces de rupture, pour être sécable au voisinage de la collerette 34' de la seringue de façon à ne pas atteindre le fond de la capacité 35. L'opérateur, après remplissage de la seringue 23 jusqu'à la graduation voulue, casse cette tige 36 et fixe la seringue sur la capacité 35. Après les injections, il retire la seringue dont le piston 29 est devenu inaccessible, ce qui évite un réemploi de l'ensemble (seringue 23, catheter 22 et aiguille 21), au bénéfice de l'aseptie des interventions ultérieures.

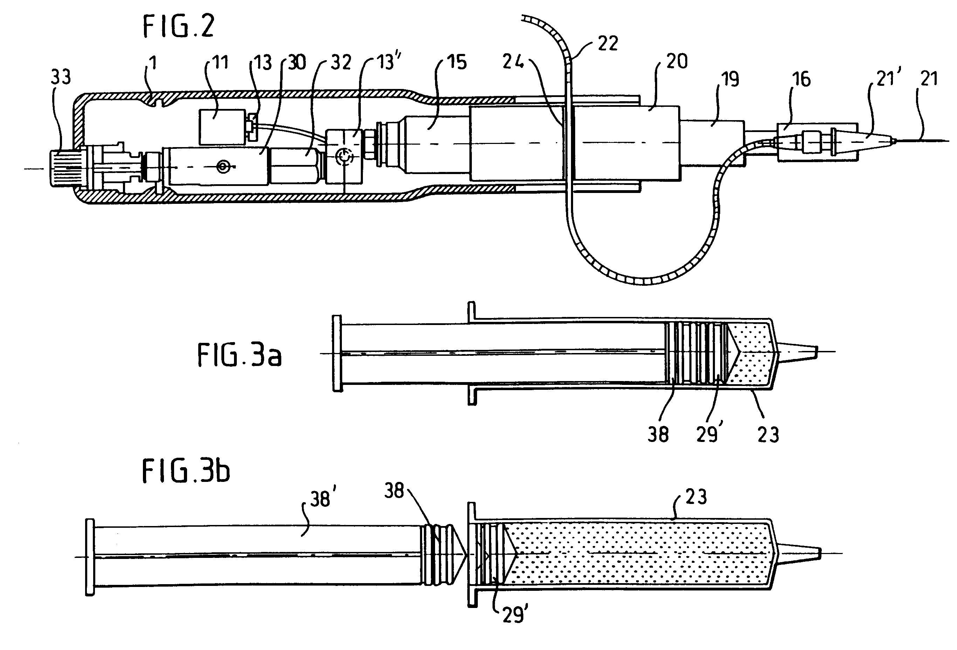

Selon une variante représentée aux figures 3a et 3b le piston 29' est libre, ne portant pas de tige. La seringue 23 est livrée avec ce piston 29' à la graduation zéro, et avec un second piston contigu 38 constituant un outillage jetable pourvu d'une tige de traction 38' (figure 3a). Par action sur celle-ci les deux pistons 29' et 38 se déplacent vers l'ouverture de la seringue jusqu'à ce que le piston d'outillage 38 débouche hors de celle-ci. Alors le piston 29' affleure sa collerette et la seringue est remplie complètement et prête à être fixée sur la capacité 35.

On note que cette capacité 35 est petite et bien plus légère qu'un vérin de même longueur que la seringue. On pourrait donc, selon une variante non représentée au dessin, fixer sur la catapulte 16 la chambre de pression 35 et donc la seringue 23, sans trop alourdir l'ensemble mobile.

On a représenté par ailleurs à la figure 1 une pièce 37 qui entoure et protège l'aiguille 21. Cette pièce 37 est échancrée à une extrémité pour laisser l'aiguille 21 apparente et guider l'opérateur quant au point de pénétration de cette aiguille. Par l'autre extrémité la pièce 37 est vissée plus ou moins sur le guide fixe 19, ce qui permet de régler la profondeur de pénétration de l'aiguille 21. Un contre écrou 39, représenté sur la figure 4, permet de bloquer cette pièce échancrée 37.

On comprendra que la description ci-dessus a été donnée à titre d'exemple, sans caractère limitatif, et que des adjonctions ou des modifications constructives pourraient y être apportées sans sortir du cadre de l'invention.

## Revendications

1. Dispositif pour injection de liquide, constitué par une pièce à main (1) contenant d'une part un vérin pneumatique (15) conçu pour pousser à l'encontre d'un ressort de rappel une catapulte (16) portant une aiguille d'injection (21) et d'autre part une seringue (23) amovible contenant le liquide et connectée à l'aiguille (21) avec en outre un organe pneumatique (35) pour pousser un piston (29) mobile dans la seringue (23), le vérin (15) et l'organe pneumatique (35) étant alimentés par un même récipient (2) équipé d'une part d'un détendeur (11) délivrant un gaz à pression constante au vérin (15) et d'autre part d'un orifice à section variable délivrant du gaz sous pression réglable à l'organe pneumatique (35), caractérisé en ce que l'orifice à section variable fait partie d'un deuxième détendeur (30) qui délivre du gaz à pression stable et réglable à l'organe pneumatique (35), et en ce que la pièce à main (1) contient le récipient (2), le détendeur à pression constante (11) et le deuxième détendeur (30).

2. Dispositif selon la revendication 1, caractérisé en ce que ledit récipient (2) constitue une poignée de la pièce à main (1).

3. Dispositif selon l'une des revendications 1 ou 2, caractérisé en ce que le récipient (2) comporte un clapet anti-retour autoclave (8), lequel est poussé de l'extérieur par un ressort (10) qui soulève le clapet (8) et vidange le récipient (2) pour toute pression interne inférieure à une pression minimale déterminée.

4. Dispositif selon l'une quelconque des revendications 1 à 3, caractérisé en ce que la pièce à main (1) comporte une vanne manuelle (14) à bouton poussoir dont la poussée assure l'alimentation du vérin (15) de poussée de la catapulte (16) et dont la libération assure la mise à l'air libre dudit vérin (15).

5. Dispositif selon la revendication 4, caractérisé en ce que la mise à l'air libre du vérin de poussée (15) se fait à travers un petit orifice (28) pour ralentir le retour de l'aiguille (21).

6. Dispositif selon l'une quelconque des revendications 1 à 5, caractérisé en ce que la catapulte (16) est portée coaxialement par le vérin pneumatique (15) et par un guide d'extrémité (18) à proximité de l'aiguille (21).

7. Dispositif selon l'une quelconque des revendications 1 à 6, caractérisé en ce qu'il comprend un moyen d'arrêt de l'écoulement de liquide sur la connexion (22) entre la seringue (23) et l'aiguille (21).

8. Dispositif selon la revendication 7, caractérisé en ce que ladite connexion est un conduit flexible (22), en ce que le moyen d'arrêt comporte une pièce de pincement (25) avec un ressort (26) qui occasionne un aplatissement du conduit (22) et en ce qu'un poussoir (27) d'ouverture écarte la pince (25), à l'encontre du ressort (26), en fin de course de la catapulte (16) poussée par le vérin (15).

9. Dispositif selon la revendication 8, caractérisé par une fente transversale (24) ménagée dans un tube fixe (20) entourant la catapulte (16), ledit tube fixe (20) contenant la pièce de pincement coulissante (25) repoussée en fin de course du vérin pneumatique (15) pour libérer du pincement le conduit flexible (22) préalablement introduit dans la fente (24).

10. Dispositif selon la revendication 9, caractérisé en ce que la pièce de pincement (25) comporte une saillie périphérique étroite (25') pour aplatir le conduit flexible (22) sur une faible surface.

11. Dispositif selon la revendication 8 ou la revendication 9, caractérisé en ce que la pièce de pincement (25) actionne une tige (40) traversant avec un faible jeu un tube fixe (20) pour aplatir le conduit (22) à l'extérieur dudit tube (20).

12. Dispositif selon l'une quelconque des revendications 1 à 11, caractérisé par un clapet anti-retour (32) interposé entre le premier détendeur (11) et l'organe pneumatique (35).

13. Dispositif selon l'une quelconque des revendications 1 à 12, caractérisé en ce que l'organe pneumatique est une chambre de pression (35) coopérant de façon étanche avec la collerette (34') de la seringue (23) pour pousser directement le piston (29) après que la seringue (23) remplie de liquide ait été fixée sur ladite chambre.

14. Dispositif selon la revendication 13, caractérisé en ce que la seringue (23) comporte un piston avec une tige (36) d'aspiration de liquide qui présente des gorges de fragilisation (36') permettant de la casser, de sorte que l'on peut jeter la partie de la tige (36) qui émerge de la seringue après son remplissage et avant qu'on la fixe sur la chambre de pression (35).

15. Dispositif selon la revendication 13, caractérisé en ce que la seringue (23) comporte un piston libre (29') et en ce que son remplissage s'effectue par traction de la tige (38') d'un piston d'outillage (38) contigü au piston libre (29') et que l'on enlève avant que l'on fixe la seringue remplie sur la chambre de pression (35).

16. Dispositif selon l'une quelconque des revendications 1 à 15, caractérisé en ce que la seringue (23), l'aiguille (21) et leur connexion (22) forment un ensemble non démontable et jetable.

17. Dispositif selon l'une quelconque des revendications 1 à 16, caractérisé par une pièce tubulaire (37) entourant et protégeant l'aiguille (21) lorsque la catapulte (16) n'est pas en action, ladite pièce (37) étant échancrée à une extrémité et emmanchée par l'autre extrémité autour d'un guide (19) de la catapulte (16).

18. Dispositif selon l'une quelconque des revendications 1 à 17, caractérisé en ce que la seringue (23) est portée par la catapulte (16), à laquelle est fixée la chambre de pression (35).

## Claims

1. Device for injection of liquid, consisting of a hand piece (1) containing on the one hand a pneumatic jack (15) designed to push a catapult (16) bearing an injection needle (21) against a return spring and on the other hand a removable syringe (23) containing the liquid and connected to the needle (21) and also with a pneumatic device (35) to push a piston (29) which is movable in the syringe (23), the jack (15) and the pneumatic device (35) being supplied by one and the same container (2) which is equipped on the one hand with a pressure reducer (11) which delivers a gas at constant pressure to the jack (15) and on the other hand with an orifice of variable cross-section which delivers gas under adjustable pressure to the pneumatic device (35), characterised in that the orifice of variable cross-section forms part of a second pressure reducer (30) which delivers gas at stable and adjustable pressure to the pneumatic device (35), and that the hand piece (1) contains the container (2), the pressure reducer at constant pressure (11) and the second pressure reducer (30).

2. Device as claimed in Claim 1, characterised in that the said container (2) forms a handle of the hand piece (1).

3. Device as claimed in one of Claims 1 or 2, characterised in that the container (2) includes an autoclave non-return valve (8) which is pushed from the exterior by a spring (10) which raises the valve (8) and drains the container (2) for any internal pressure lower than a predetermined minimum pressure.

4. Device as claimed in any one of Claims 1 to 3, characterised in that the hand piece (1) includes a push-button manual valve (14), pushing of which ensures the supply of the jack (15) for pushing the catapult (16) and release of which ensures the exposure to the open air of the said jack (15).

5. Device as claimed in Claim 4, characterised in that the exposure of the pushing jack (15) to the open air is accomplished through a small orifice (28) in order to slow down the return of the needle (21).

6. Device as claimed in any one of Claims 1 to 5, characterised in that the catapult (16) is borne coaxially by the pneumatic jack (15) and by an end guide (18) near the needle (21).

7. Device as claimed in any one of Claims 1 to 6, characterised in that it comprises a means for stopping the flow of liquid over the connection (22) between the syringe (23) and the needle (21).

8. Device as claimed in Claim 7, characterised in that the said connection is a flexible conduit (22), and that the stopping means include a gripping piece (25) with a spring (26) which brings about a flattening of the conduit (22), and that an opening pusher (27) parts the gripper (25), against the spring (26), at the end of the travel of the catapult (16) pushed by the jack (15).

9. Device as claimed in Claim 8, characterised by a transverse slot (24) produced in a fixed tube (20) surrounding the catapult (16), the said fixed tube (20) containing the sliding gripping piece (25) which is pushed back at the end of the travel of the pneumatic jack (15) in order to release from gripping the flexible conduit (22) which was previously introduced into the slot (24).

10. Device as claimed in Claim 9, characterised in that the gripping piece (25) includes a narrow peripheral projection (25') in order to flatten the flexible conduit (22) over a weak surface.

11. Device as claimed in Claim 8 or Claim 9, characterised in that the gripping piece (25) actuates a rod (40) passing with a small clearance through a fixed tube (20) in order to flatten the conduit (22) outside the said tube (20).

12. Device as claimed in any one of Claims 1 to 11, characterised by a non-return valve (32) interposed between the first pressure reducer (11) and the pneumatic device (35).

13. Device as claimed in any one of Claims 1 to 12, characterised in that the pneumatic device is a pressure chamber (35) which co-operates in a sealed manner with the flange (34') of the syringe (23) for direct pushing of the piston (29) after the syringe (23) filled with liquid has been fixed on the said chamber.

14. Device as claimed in Claim 13, characterised in that the syringe (23) includes a piston with a rod (36) for drawing in liquid which has weak necked areas (36') which allow it to be broken, in such a way that it is possible to throw away the part of the rod (36) which emerges from the syringe after it has been filled and before it is fixed on the pressure chamber (35).

15. Device as claimed in Claim 13, characterised in that the syringe (23) includes a free piston (29'), and that filling thereof is effected by pulling of the rod (38') of a piston of the assembly (38) which is contiguous with the free piston (29') and is removed before the filled syringe is fixed on the pressure chamber (35).

16. Device as claimed in any one of Claims 1 to 15, characterised in that the syringe (23), the needle (21) and the connection (22) thereof form a disposable assembly which cannot be dismantled.

17. Device as claimed in any one of Claims 1 to 16, characterised by a tubular piece (37) surrounding and protecting the needle (21) when the catapult (16) is not in action, the said piece (37) being indented at one end and fitted by the other end around a guide (19) of the catapult (16).

18. Device as claimed in any one of Claims 1 to 17, characterised in that the syringe (23) is borne by the catapult (16) to which the pressure chamber (35) is fixed.

## Patentansprüche

1. Vorrichtung zur Injektion einer Flüssigkeit, bestehend aus einem Handstück (1), enthaltend einerseits eine pneumatische Druckvorrichtung (15), vorgesehen um entgegen einer Rückholfeder ein eine Injektionsnadel (21) tragendes Katapult (16) zu stoßen, und andererseits eine auswechselbare Spritze (23), beinhaltend die Flüssigkeit und verbunden mit der Nadel (21), und überdies ein pneumatisches Organ (35), um einen in der Spritze (23) beweglichen Kolben (29) zu stoßen; die Druckvorrichtung (15) und das pneumatische Organ (35) sind durch dasselbe Behältnis (2) versorgbar, versehen einerseits mit einem Druckreduzierer (11), der der Druckvorrichtung (15) ein Gas mit konstantem Druck liefert und andererseits mit einer Mündung mit variablem Querschnitt, mittels der dem pneumatischen Organ (35) Gas unter regelbarem Druck zulieferbar ist,
**dadurch gekennzeichnet,**
daß die Mündung mit variablem Querschnitt ein Teil eines zweiten Druckreduzierers (30) ausmacht, der das pneumatische Organ (35) mit Gas bei stetigem und regulierbarem Druck beliefert und dadurch, daß das Handstück (1) das Behältnis (2), den konstanten Druckreduzierer (11) und den zweiten Druckreduzierer (30) umfaßt.

2. Vorrichtung nach Anspruch 1, dadurch gekennzeichnet, daß das besagte Behältnis (2) einen Handgriff des Handstücks (11) bildet.

3. Vorrichtung nach einem der Ansprüche 1 oder 2, dadurch gekennzeichnet, daß das Behältnis (2) eine autoklave Rückschlagklappe (8) trägt, die von außen von einer Feder (10) gestoßen ist, die die Klappe (8) anhebt und das Behältnis (2) für den gesamten inneren Druck bis auf einen bestimmten minimalen Druck entleert.

4. Vorrichtung nach einem der Ansprüche 1 bis 3, dadurch gekennzeichnet, daß das Handstück (1) ein durch Druckknopf manuell betätigbares Ventil (14) enthält, dessen Betätigung die Versorgung der das Katapult (16) stoßenden Druckvorrichtung (15) sichert und dessen Freigabe die Umstellung der angesprochenen Druckvorrichtung (15) auf die freie Luft sichert.

5. Vorrichtung nach Anspruch 4, dadurch gekennzeichnet, daß die Umstellung der stoßenden Druckvorrichtung (15) auf die freie Luft sich bei Durchqueren einer kleinen Öffnung (28) ereignet, um das Zurückkommen der Nadel (21) zu dämpfen.

6. Vorrichtung nach einem der Ansprüche 1 bis 5, dadurch gekennzeichnet, daß das Katapult (16) koaxial durch die Druckvorrichtung (15) und durch eine Außenführung (18) nahe der Injektionsnadel (21) getragen ist.

7. Vorrichtung nach einem der Ansprüche 1 bis 6, dadurch gekennzeichnet, daß sie ein Mittel zum Verhindern des Ausleckens der Flüssigkeit auf der Verbindung (22) zwischen der Spritze (23) und der Nadel (21) enthält.

8. Vorrichtung nach Anspruch 7, dadurch gekennzeichnet, daß die besagte Verbindung eine flexible Leitung (22) ist, dadurch daß das Mittel zum Verhindern ein Klemmstück (25) mit einer Rückholfeder (26) trägt, das eine Abplattung der Leitung (22) ermöglicht und dadurch, daß ein Öffnungsstößel (27) die Klemme (25) spreizt, entgegen der Rückholfeder (26), am Ende des Weges des durch die Druckvorrichtung (15) gestoßenen Katapultes (16).

9. Vorrichtung nach Anspruch 8, gekennzeichnet durch einen Querspalt (24), angebracht in einem das Katapult (16) umgebenden Festrohr (20), besagtes Festrohr (20) enthaltend das bewegliche Klemmstück (25), das am Ende des Weges der pneumatischen Druckvorrichtung (15) zurückgestoßen wird, um die Klemmung der flexiblen Leitung (22) freizugeben, welche vorher in den Spalt (24) eingeführt ist.

10. Vorrichtung nach Anspruch 9, dadurch gekennzeichnet, daß das Klemmstück (25) eine schmale, ringförmige Lippe (25') umfaßt für die Abplattung der flexiblen Leitung (22) auf eine geringe Oberfläche.

11. Vorrichtung nach Anspruch 8 oder 9, dadurch gekennzeichnet, daß das Klemmstück (25) einen Bolzen (40) betätigt, der mit geringem Spiel das Festrohr (20) durchdringt, um die Leitung (22) an der Außenseite des besagten Rohres (20) abzuplatten.

12. Vorrichtung nach einem der Ansprüche 1 bis 11, gekennzeichnet durch eine zwischen den ersten Druckreduzierer (11) und das pneumatische Organ (35) gesetzte Rückschlagklappe (32).

13. Vorrichtung nach einem der Ansprüche 1 bis 12, dadurch gekennzeichnet, daß das pneumatische Organ eine Druckkammer (35) ist, die in abdichtender Form mit der Dichtung (34') der Spritze (23) zusammenwirkt, um direkt den Kolben (29) zu stoßen, nachdem die mit Flüssigkeit gefüllte Spritze (23) an der besagten Kammer fixiert ist.

14. Vorrichtung nach Anspruch 13, dadurch gekennzeichnet, daß sich an der Spritze (23) ein Kolben mit einer Stange (36) für das Ansaugen der Flüssigkeit befindet, die Sollbruchstellen aufweist, die das Brechen in einer Art erlauben, daß man das Teil der Stange (36) abstoßen kann, welches an der Spritze nach ihrer Füllung hervorragt, bevor man sie auf der Druckkammer (35) befestigt.

15. Vorrichtung nach Anspruch 13, dadurch gekennzeichnet, daß die Spritze (23) einen freien Kolben (29') umfaßt und dadurch, daß ihre Füllung bewirkt wird durch Zug der Kolbenstange (38') eines zum freien Kolben (29') benachbarten Nutzkolbens (38), und den man abnimmt, bevor man die gefüllte Spritze auf der Druckkammer (35) fixiert.

16. Vorrichtung nach einem der Ansprüche 1 bis 15, dadurch gekennzeichnet, daß die Spritze (23), die Nadel (21) und ihre Verbindung (22) eine nicht zerlegbare und wegwerfbare Einheit bilden.

17. Vorrichtung nach einem der Ansprüche 1 bis 16, gekennzeichnet durch ein rohrartiges Teil (37), die Nadel (21) umhüllend und schützend, wenn das Katapult (16) nicht in Aktion ist, besagtes Element (37) ist an einem Ende ausgebuchtet und an dem anderen Ende um eine Führung (19) des Katapultes (16) setzbar.

18. Vorrichtung nach einem der Ansprüche 1 bis 17, dadurch gekennzeichnet, daß die Spritze (23) von dem Katapult (16) getragen ist, an welchem die Druckkammer (35) fixiert ist.
